(19) Europäisches Patentamt
European Patent Office
Office européen des brevets

(11) EP 3 512 486 B2

(12) NEW EUROPEAN PATENT SPECIFICATION

After opposition procedure

(45) Date of publication and mention of the opposition decision:
**12.07.2023 Bulletin 2023/28**

(45) Mention of the grant of the patent:
**21.10.2020 Bulletin 2020/43**

(21) Application number: **17761527.5**

(22) Date of filing: **06.09.2017**

(51) International Patent Classification (IPC):
***A61K 8/02*** (2006.01) ***A61K 8/26*** (2006.01)
***A61K 8/27*** (2006.01) ***A61K 8/43*** (2006.01)
***A61K 8/44*** (2006.01) ***A61K 8/49*** (2006.01)
***A61K 8/60*** (2006.01) ***A61K 8/67*** (2006.01)
***A61K 8/88*** (2006.01) ***A61Q 1/10*** (2006.01)

(52) Cooperative Patent Classification (CPC):
**A61K 8/022; A61K 8/26; A61K 8/27; A61K 8/43; A61K 8/44; A61K 8/442; A61K 8/4946; A61K 8/4953; A61K 8/602; A61K 8/606; A61K 8/673; A61K 8/88; A61Q 1/10**

(86) International application number:
**PCT/EP2017/072304**

(87) International publication number:
**WO 2018/050504 (22.03.2018 Gazette 2018/12)**

# (54) BLUE POWDER AND USE THEREOF IN COSMETICS

BLAUES PULVER UND VERWENDUNG DAVON IN DER KOSMETIK

POUDRE BLEUE ET SON UTILISATION EN COSMÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.09.2016 FR 1658683**

(43) Date of publication of application:
**24.07.2019 Bulletin 2019/30**

(73) Proprietor: **L'OREAL**
**75008 Paris (FR)**

(72) Inventors:
- **CHOISY, Patrick**
  **37270 Montlouis Sur Loire (FR)**
- **GUENAULT, Emilie**
  **37097 TOURS Cedex 2 (FR)**

(74) Representative: **L'Oreal**
**Service D.I.P.I.**
**9, rue Pierre Dreyfus**
**92110 Clichy (FR)**

(56) References cited:
**WO-A1-2014/029842**

- **GREGORY T. SIGURDSON ET AL: "Bathochromic and Hyperchromic Effects of Aluminum Salt Complexation by Anthocyanins from Edible Sources for Blue Color Development", JOURNAL OF AGRICULTURAL AND FOOD CHEMISTRY, vol. 62, no. 29, 23 July 2014 (2014-07-23) , pages 6955-6965, XP055352726, US ISSN: 0021-8561, DOI: 10.1021/jf405145r**
- **BROUILLARD R ET AL: "pH and Solvent Effects on the Copigmentation Reactionof Malvin with Polyphenols Purine and Pyrimidine Derivatives", JOURNAL OF THE CHEMICAL SOCIETY, PERKIN TRANSACTIONS 2, CHEMICAL SOCIETY. LETCHWORTH, GB, vol. 8, 1 January 1991 (1991-01-01), pages 1235-1241, XP009156684, ISSN: 1472-779X**

EP 3 512 486 B2

## Description

The present invention relates to a coloured powder comprising an anthocyanin, a composition containing the coloured powder and a process for treating keratin materials using the coloured powder.

For several years, there has been an increasing interest in natural compounds of use as coloured material, especially in the field of cosmetics.

The colour range available in natural dyes or in natural pigments is not as broad as that of synthetic dyes or synthetic pigments, and many natural dyes or natural pigments have poor stability during their exposure to light. In particular, there are very few natural blue dyes or natural blue pigments, and they are not sufficiently photostable: their hue fades and thus does not produce a very attractive colour effect.

Coloured blue powders obtained from anthocyanins, metal ions derived from Al(III), Ca(II), Fe(II), Mg(II), Zn(II), and tannic acid or its gallotannin derivatives, used for making up keratin materials, are known from document WO2014/029842. These coloured powders have good photostability. Nonetheless, the blue colour of these coloured powders is not very vibrant and therefore blue powders are sought which have better chromaticity, in order to be able to obtain better colour effects, especially with the makeup products containing such powders.

The aim of the present invention is thus to make available a blue coloured powder having a good hue angle, good chromaticity and also good photostability.

The inventors have discovered that such a coloured powder is obtained by stabilizing anthocyanins with certain complexed metals and certain nitrogen-based compounds, as defined below.

Aside from the good properties of chromaticity and photostability, the powders according to the invention also have at least one good property from properties of coverage, tinting strength and lightness.

More specifically, the invention relates to a coloured, water-insoluble powder comprising the combination of an anthocyanin, a complexed metal chosen from aluminium or zinc, and a particular nitrogen-based compound as described below.

The invention also relates to a process for preparing the coloured, water-insoluble powder.

The invention also relates to a composition comprising, in a physiologically acceptable medium, a coloured, water-insoluble powder as described previously.

The invention also relates to a process for treating keratin materials, comprising the application, to the keratin materials, of a coloured, water-insoluble powder as described above or of a composition containing same.

The process is advantageously carried out on human keratin materials.

The process for treating keratin materials is preferably a process for making up keratin materials, in particular human keratin materials.

Within the meaning of the present invention, keratin materials is intended to mean the skin (especially the skin of the face, the cheeks and the eyelids), the lips, the hair, the nails, the eyelashes and the eyebrows.

Water-insoluble powder is intended to mean a pulverulent material (hence in particulate form) which is not soluble in water or ethanol or mixtures of water/ethanol in any proportions, at a concentration of 0.5% by weight and at room temperature (25°C).

The document "Le mystère de la couleur des fleurs, des fruits ... et du vin!" ("The mystery of the colour of flowers, fruit...and wine!") by Raymond Brouillard and Andre Fougerosse, dated 18 February 2008 (URL: http://radium.net.espci.fr/esp/CONF/2008/C08_02/conf02_2008.htm) describes that a mixture of blueberry extract, aluminium nitrate and caffeine in water gives a blue-coloured solution. This blue aqueous solution appears clear, without the formation of a precipitate: the formation of water-insoluble coloured particles is not demonstrated in this mixture produced.

The coloured powder according to the invention comprises at least one anthocyanin.

As is known, anthocyanins are anthocyanidin glycosides (Jin-Ming Kong et al., Phytocomposé, 64, 923-933 (2003)).

There are more than 500 different anthocyanins in nature. Their main differences relate to the number of hydroxyl groups and methoxy groups, the nature and the number of sugar units present, the aliphatic or aromatic carboxylate groups bonded to the sugars in the molecule, and the position of these bonds (A Castañeda-Ovando et al., Food Compose, 113, 859-871 (2009)).

Anthocyanidins are compounds of formula (I) below:

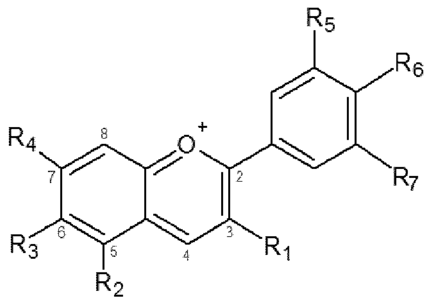

(I)

in which the radicals $R_1$ to $R_7$ denote, independently, H, OH or OMe, it being understood that at least one of the radicals $R_1$ to $R_7$ denotes a sugar radical.

The 6 most common anthocyanidins are indicated in Table 1 below, with their name, abbreviation and substituents of formula (I) described above.

Table 1: common anthocyanidins

| Name (abbreviation) | $R_1$ | $R_2$ | $R_3$ | $R_4$ | $R_5$ | $R_6$ | $R_7$ |
|---|---|---|---|---|---|---|---|
| Cyanidin (Cy) | OH | OH | H | OH | OH | OH | H |
| Delphinidin (Dp) | OH | OH | H | OH | OH | OH | OH |
| Pelargonidin (Pg) | OH | OH | H | OH | H | OH | H |
| Peonidin (Pn) | OH | OH | H | OH | OMe | OH | H |
| Petunidin (Pt) | OH | OH | H | OH | OMe | OH | OH |
| Malvidin (Mv) | OH | OH | H | OH | OMe | OH | OMe |

As indicated above, anthocyanins may contain sugar units chosen from glucose, rhamnose, galactose, xylose, arabinose, rutinose and glucuronic acid. The sugar units may be present in the form of monosaccharide or oligosaccharides, such as, for example, diglucose.

Most anthocyanins in nature are 3-monosaccharides or 3,5-disaccharides such as 3,5-diglucosides and 3,7-diglucosides. Anthocyanins may also be acylated: the sugar units may be acylated by esterification with a carboxylic acid, especially having 2 to 15 carbon atoms, such as acetic acid, malonic acid, p-coumaric acid, ferulic acid, sinapic acid or caffeic acid.

Advantageously, the anthocyanins present in the coloured, water-insoluble powder according to the invention may be chosen from those of formula (I'):

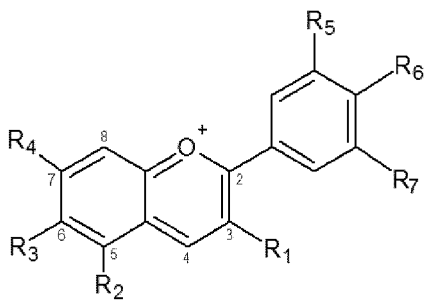

(I')

in which $R_1$, $R_2$ and $R_4$ independently denote H, OH, OMe, a sugar unit or an acylated sugar unit (acyl group having especially from 2 to 15 carbon atoms, such as those derived from acetic acid, malonic acid, p-coumaric acid, sinapic acid, ferulic acid or caffeic acid), it being understood that at least one of the radicals $R_1$, $R_2$ and $R_4$ denotes a sugar unit or an acylated sugar unit, and $R_3$, $R_5$, $R_6$ and $R_7$ independently denote H, OH or OMe.

The anthocyanins may be of natural or synthetic origin.

A certain number of plants are naturally rich in anthocyanins. Mention may be made, by way of example, of black carrot, elderberry, hibiscus, blackcurrant, purple corn, black potato, red cabbage, red onion, purple potato, black grape, cranberry, strawberry, raspberry, aronia, black soybean, radish, gooseberry, blueberry, cherry, aubergine and black rice.

The anthocyanins used according to the present invention may be chosen from anthocyanins, the anthocyanidin-derived units of which are chosen from cyanidin, delphinidin, malvidin, peonidin or petunidin, the sugar(s) present preferably being chosen from galactose, glucose, arabinose, rutinose or diglucose, this (these) sugar(s) possibly being acylated by acetic acid, malonic acid, p-coumaric acid, sinapic acid, ferulic acid or caffeic acid.

Thus, the anthocyanins used according to the invention may be chosen from:

cyanidin-3-O-arabinoside, cyanidin-3-O-galactoside, cyanidin-3-O-glucoside, cyanidin-3,5-O-diglucoside, cyanidin-3-O-rutinoside,
delphinidin-3-O-arabinoside, delphinidin-3-O-galactoside, delphinidin-3-O-glucoside, delphinidin-3-O-glucoside, delphinidin-3-O-rutinoside,
malvidin-3-O-arabinoside, malvidin-3-O-galactoside, malvidin-3-O-glucoside, malvidin-3,5,-O-diglucoside, peonidin-3-O-glucoside, peonidin-3-O-glucoside,
petunidin-3-O-arabinoside, petunidin-3-O-galactoside, petunidin-3-O-glucoside,
these anthocyanins possibly being acylated by acetic acid, malonic acid, p-coumaric acid, sinapic acid, ferulic acid or caffeic acid.

The four predominant anthocyanins present in blackcurrants are cyanidin-3-O-glucoside, cyanidin-3-O-rutinoside, delphinidin-3-O-glucoside and delphinidin-3-O-rutinoside. Black grapes and red cabbage are the two largest sources of anthocyanins in nature. The anthocyanidin units of the anthocyanins present in black grapes are cyanidin, peonidin, malvidin, petunidin and delphinidin; and the organic acids present are acetic, coumaric and caffeic acids. The only sugar present is glucose (F.J. Francis, Colorants, p56, Eagan Press (1999)). One grape anthocyanin, malvidin-3,5,-diglucoside, corresponds to the following formula (II), in which Glu is glucose and Me is the methyl radical.

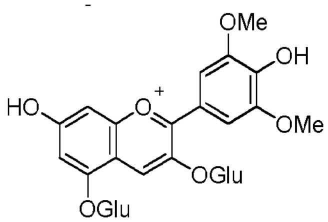

(II)

According to one embodiment of the invention, the anthocyanins used may be chosen from cyanidin-3-O-galactoside, cyanidin-3-O-glucoside, cyanidin-3-O-arabinoside, delphinidin-3-O-galactoside, delphinidin-3-O-glucoside, delphinidin-3-O-arabinoside, malvidin-3-O-galactoside, malvidin-3-O-glucoside, malvidin-3-O-arabinoside, peonidin-3-O-glucoside, petunidin-3-O-galactoside, petunidin-3-O-glucoside, petunidin-3-O-arabinoside.

According to another embodiment of the invention, the anthocyanins used may be chosen from cyanidin-3-O-glucoside, peonidin-3-O-glucoside, cyanidin-3,5-O-diglucoside, cyanidin-3-rutinoside.

Seven of the anthocyanins present in red cabbage are described in formula (III) and Table 2 below. The $R_1$ and $R_2$ groups of these seven anthocyanins are indicated in Table 2.

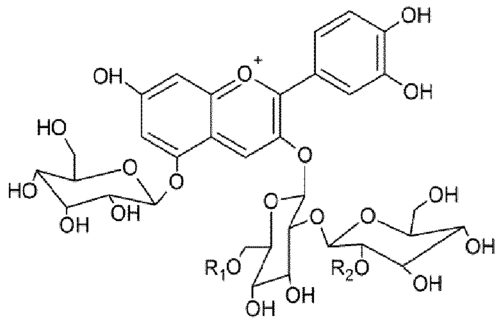

(III)

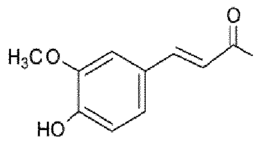

Feruloyl

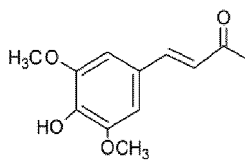

Sinapoyl

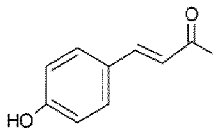

p-Coumaroyl

Table 2

| | $R_1$ | $R_2$ |
|---|---|---|
| A | H | Sinapoyl |
| B | Sinapoyl | H |
| C | Feruloyl | H |
| D | p-Coumaroyl | H |
| E | Sinapoyl | Sinapoyl |
| F | Feruloyl | Sinapoyl |
| G | p-Coumaroyl | Sinapoyl |

The anthocyanins used according to the present invention may be one or more of the cyanidin-3-diglucosides A to G described above in Table 2.

The anthocyanins may be added in the form of plant material or of an extract of plant material.

The plant material may be chosen, for example, from vegetables, fruit or flowers. The plant material may be chosen from red cabbage, red onion, purple potato, grape, cranberry, strawberry, raspberry, aronia, black soybean, blackcurrant, elderberry, hibiscus, radish, gooseberry, blueberry, cherry, aubergine, black carrot and black rice.

In particular, the anthocyanins present in the coloured, water-insoluble powder may be derived from blueberry or a blueberry extract.

Blueberry is intended to mean *Vaccinium sp*, for example *Vaccinium myrtillus,* common bilberry, *Vaccinium uliginosum,* bog blueberry, or also *Vaccinium caespitosum, Vaccinium corymbosum*, *Vaccinium angustifolium, Vaccinium deliciosum, Vaccinium membranaceum*, *Vaccinium ovalifolium*, or *Vaccinium myrtilloides. Vaccinium myrtillus* is preferably used.

In particular, the anthocyanins present in the coloured, water-insoluble powder may be derived from black rice or a black rice extract.

Rice is intended to mean Oryza sp, for example *Oryza sativa,* rice originating from the wet tropics of Asia, and *Oryza glaberrima*, West African rice, which are the two species of cultivated rice, or *Oryza sativa L. indica*, *or Oryza sativa L. japonica*, the subspecies of Asian rice.

The anthocyanin may be present in the coloured, water-insoluble powder in a content ranging from 0.05% to 50% by weight, relative to the total weight of dry matter of the coloured powder, preferably ranging from 0.3% to 30% by weight, preferentially ranging from 5% to 30% by weight, and more preferentially ranging from 10% to 30% by weight.

Metal ions

The coloured powder according to the invention comprises a complexed metal chosen from aluminium or zinc or the mixture thereof.

The complexed metal is especially formed from metal ions chosen from $Al^{3+}$, $Zn^{2+}$ and mixtures thereof. These metal ions make it possible to stabilize the anthocyanins by complex formation. The complexed metal is advantageously derived from aluminium or zinc metal ions and in particular from aluminium or zinc salts formed with anions such as gluconate, chloride, sulfate and acetate.
For example, such salts may be chosen from aluminium sulfate, $Al_2(SO_4)_3$, containing the ($Al^{3+}$) ion, ammonium aluminium sulfate, aluminium acetate, aluminium chloride, aluminium chlorohydrate, zinc acetate, zinc gluconate, zinc chloride, zinc sulfate, $Zn(SO_4)$ containing the ($Zn^{2+}$) ion, and mixtures thereof.

The complexed metal is preferably derived from salts chosen from ammonium aluminium sulfate, aluminium chloride, zinc sulfate, and mixtures thereof.

The complexed metal may be present in the coloured powder at a weight ratio of metal ions/anthocyanin ranging from 0.01/1 to 10/1, preferably ranging from 0.05/1 to 5/1.

Nitrogen-based compound:

The coloured powder according to the invention comprises a nitrogen-based compound chosen from nitrogen-based heterocyclic compounds of formula (IVa) or of formula (IVb) or nitrogen-based compounds of formula (Va) or amino polymers as described below.

The nitrogen-based compound may be a heterocyclic nitrogen-based compound of formula (IVa):

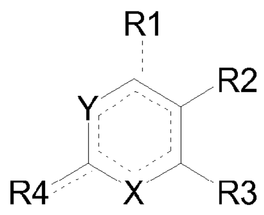
(IVa)

in which:

the --- bonds may signify a single bond or a double bond, it being understood that 2 contiguous bonds cannot be double bonds, the nitrogen-based heterocycles being able to be saturated, unsaturated or aromatic;

X denotes $-C(R_5)=$ or $-N=$ or $-N(R_6)-$ ;

Y denotes $-N=$ or $-N(R_7)-$ or $-(R_8)N^+-$, $An^-$

$R_1$ denotes an amino radical, a (C1-C4)alkylamino radical, a (C1-C4)dialkylamino radical, an oxygen atom (in carbonyl form), a hydrogen atom, or a C1-C4 alkyl radical, optionally substituted by at least one hydroxyl group, such as methyl;

$R_2$ denotes a hydrogen atom, a hydroxyl radical, a carboxyl ($-CO_2H$) radical, or a C1-C4 alkyl radical such as methyl;

$R_3$ denotes a hydrogen atom or a C1-C4 alkyl radical optionally substituted by one or more hydroxyl radicals, such as hydroxymethyl;

$R_4$ denotes a hydrogen atom, an amino radical or an oxygen atom (in carbonyl form);

$R_5$ denotes a hydrogen atom or a C1-C4 alkyl radical optionally substituted by one or more hydroxyl radicals, such as hydroxymethyl;

$R_6$ denotes a hydrogen atom, a C1-C4 alkyl radical such as methyl, or a radical of formula (aa):

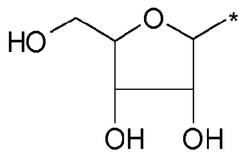

(aa)

$R_7$ denotes a hydrogen atom or a C1-C4 alkyl radical such as methyl;

$R_8$ denotes a C1-C4 alkyl radical such as methyl;

$R_2$ and $R_3$ possibly forming, together with the carbon atoms which bear them, a saturated or unsaturated 4- to 8-membered (hetero)cycle, 1 or more non-adjacent ring members of which may denote a nitrogen atom -N= or an -N($R_9$)- radical, with $R_9$ denoting a hydrogen atom or a radical $R_{10}$ of formula:

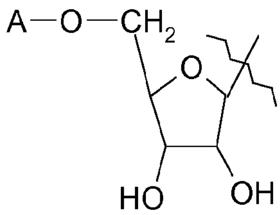

**($R_{10}$)**

in which A is chosen from a hydrogen atom, or a (dd), (ee) or (ff) radical as follows:

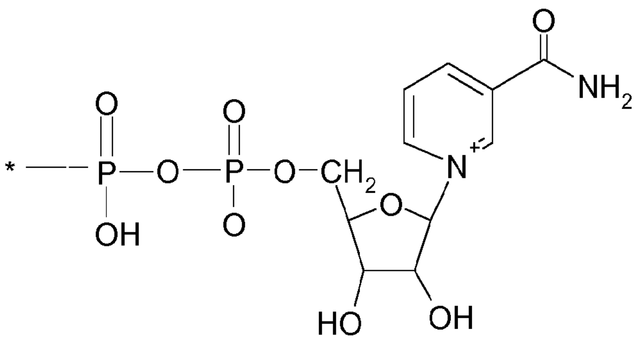

(dd)

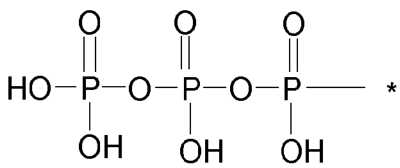

(ee)

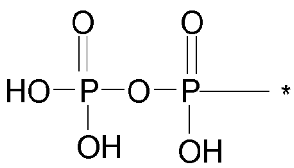

(ff)

* indicates the point of attachment of the radical A to the oxygen atom of R10

An$^-$ denotes a cosmetically acceptable anion such as a halide anion, such as, for example, a chloride anion; and the salts thereof.

Preferably, when $R_2$ and $R_3$ form, together with the carbon atoms which bear them, a saturated or unsaturated

(hetero)cycle, this heterocycle comprises 5 or 6 ring members, preferably 5 ring members, and one or more non-adjacent ring members denote a nitrogen atom -N= or an -N($R_9$)- radical as defined above.

Particularly preferably, $R_2$ and $R_3$ form, together with the carbon atoms which bear them, a 5- or 6-membered, preferably 5-membered, unsaturated heterocycle, two non-adjacent ring members of which denote, on the one hand, a nitrogen atom -N= and, on the other hand, an -N($R_9$)- radical as defined above.

By way of example of compound (IVa), mention may be made of:

| Nitrogen-based compound | CAS no. | Chemical structure |
|---|---|---|
| (a) Nicotinamide Adenine Diphosphate | 53-84-9 | 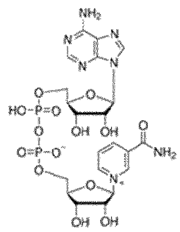 |
| (b) Pyridoxine | 65-23-6 | 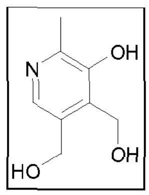 |
| (c) Adenosine triphosphate, disodium salt | 987-65-5 | 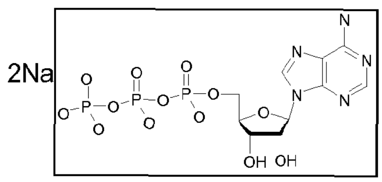 |
| (d) Adenosine Diphosphate | 58-64-0 | 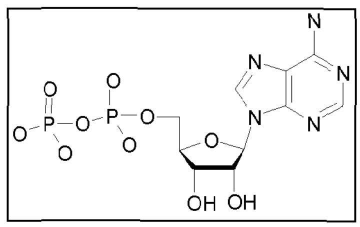 |
| (e) Theophylline | 58-55-9 | 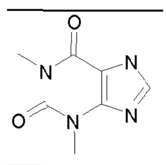 |
| (f) Theobromine | 83-67-0 | 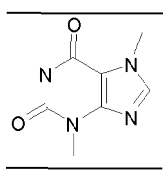 |

(continued)

| Nitrogen-based compound | CAS no. | Chemical structure |
|---|---|---|
| (g) Adenosine | 58-61-7 | 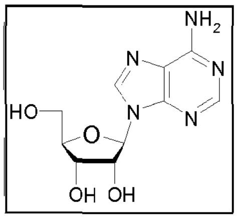 |
| (h) Thymidine | 65-71-4 | 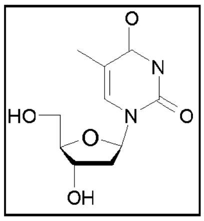 |
| (i) Guanosine | 118-00-3 | 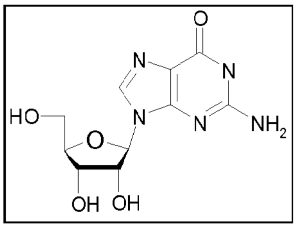 |
| (j) Guanine | 73-40-5 | 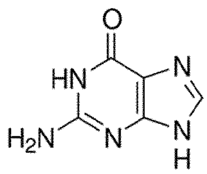 |
| (k) Cytidine | 65-46-3 | 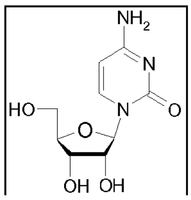 |
| (l) Uridine | 58-96-8 | 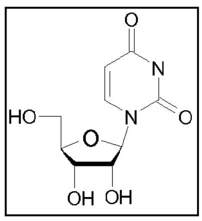 |
| (m) Caffeine | 58-08-2 | 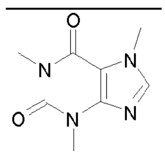 |

(continued)

| Nitrogen-based compound | CAS no. | Chemical structure |
|---|---|---|
| (n) Xanthine | 69-89-6 | 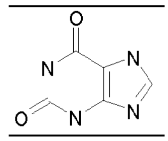 |
| (o) Nicotinic acid | 59-67-6 | 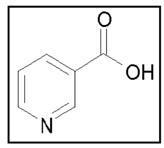 |
| (p) Trigonelline | 6138-41-6 | 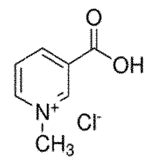 |
| (q) Paraxanthine | 611-59-6 | 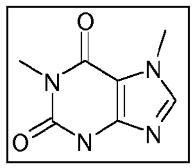 |
| (r) Adenine | 73-24-5 | 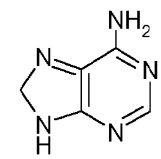 |

The compound (IVa) is preferably chosen from nicotinamide adenine diphosphate, adenosine triphosphate disodium salt, adenosine diphosphate, theobromine, adenosine, thymidine, guanine, cytidine, uridine, caffeine, xanthine, nicotinic acid, trigonelline, paraxanthine, and adenine.

The nitrogen-based compound may be a heterocyclic nitrogen-based compound of formula (IVb):

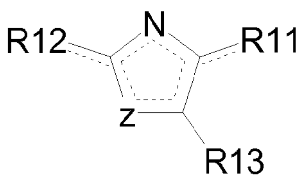

(IVb)

in which:

the --- bonds may signify a single bond or a double bond, it being understood that 2 contiguous bonds cannot be double bonds, the nitrogen-based heterocycles being able to be saturated, unsaturated or aromatic;

$R_{11}$ denotes an oxygen atom (in carbonyl form) or an $-CH_2-CH(NH_2)-CO_2H$ radical or a -COOH group;

$R_{12}$ denotes a hydrogen atom or an oxygen atom (in carbonyl form);

$R_{13}$ denotes a hydrogen atom or an $-NH-CO-NH_2$ radical;

Z denotes a nitrogen atom -N= or an-N($R_{14}$)- radical, in which $R_{14}$ denotes a hydrogen atom or a -$CH_2$-CH($NH_2$)-$CO_2H$ radical, or a -CH2- radical; and the salts thereof.

When the compound (IVa) or (IVb) comprises an anionic group such as a carboxylic acid group, the anionic group may be in salt form, for example in the form of an alkali metal salt or alkaline earth metal salt, such as sodium, potassium, magnesium or calcium salt.

By way of example of compound (IVb), mention may be made of:

| Nitrogen-based compound | CAS no. | Chemical structure |
|---|---|---|
| (s) Histidine | 71-00-1 | 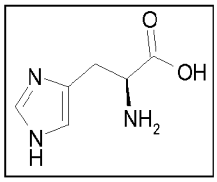 |
| (t) Allantoin | 97-59-6 | 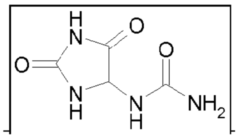 |
| (u) Tryptophan | 54-12-6 | 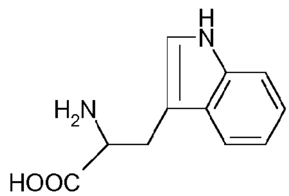 |
| (v) proline | 147-85-3 | 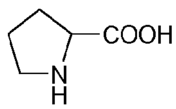 |

The heterocyclic nitrogen-based compound (IVa) or (IVb) is preferably chosen from nicotinamide adenine diphosphate, adenosine triphosphate disodium salt, adenosine diphosphate, theobromine, adenosine, thymidine, guanine, cytidine, uridine, caffeine, xanthine, nicotinic acid, trigonelline, paraxanthine, adenine, histidine, allantoin, tryptophan and proline.

The heterocyclic nitrogen-based compound is preferentially chosen from pyridoxine, adenosine triphosphate disodium salt, adenosine, caffeine, allantoin, trigonelline, histidine and adenine.

The heterocyclic nitrogen-based compound is more preferentially chosen from pyridoxine, adenosine triphosphate disodium salt, adenosine, allantoin, trigonelline, histidine and adenine.

The nitrogen-based compound may be a compound of formula (Va):

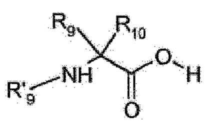

(Va)

in which:

- Rg represents a hydrogen atom or a linear or branched $C_1$-$C_6$ alkyl radical, optionally substituted by one or more identical or different groups chosen from hydroxyl, hydroxycarbonyl, thiol, ($C_1$-$C_4$)alkylthio, amido, amino or guanidine radicals, or a phenyl radical, optionally substituted by one or more hydroxyls;
- R'g represents a hydrogen, a $C_1$-$C_4$ alkyl radical, or an unsubstituted benzyl radical, or a $C_2$-$C_{16}$ acyl radical; R'g

preferably represents a hydrogen atom;

- $R_{10}$ represents a hydrogen or a $C_1$-$C_4$ alkyl radical; $R_{10}$ preferably represents a hydrogen atom.

and the salts thereof.

Among the groups present as substituents of the heterocyclic hydrocarbon-based groups, mention may be made of the following groups:

carboxylic, in acid or salified form,
sulfonic, in acid or salified form,
phosphonic, in acid or salified form,
hydroxyl,
$C_1$-$C_4$ alkoxy,
($C_1$-$C_8$)alkoxycarbonyl,
($C_1$-$C_4$)alkylsulfonate,
($C_1$-$C_8$)alkyl phosphonate,
($C_1$-$C_4$)trialkylsilyl,
($C_1$-$C_4$)trialkoxysilanyl,
amino;
(di)($C_1$-$C_4$)alkylamino,
($C_1$-$C_4$)trialkylammonium,
thiol,
($C_1$-$C_4$)alkylthio,
aminosulfonyl,
(di-)($C_1$-$C_4$)alkylaminosulfonyl,
aminocarbonyl,
(di)($C_1$-$C_4$)alkylaminocarbonyl,
($C_1$-$C_4$)alkylcarbonylamino,
guanidine
ureido ($N(R)_2$-CO-NR'-) in which the radicals R and R', independently of one another, represent a hydrogen atom, a $C_1$-$C_4$ alkyl radical or a ($C_1$-$C_4$)alkylsulfonylamino radical;
phenyl, optionally substituted by one or more $C_1$-$C_2$ alkyl or hydroxyl.

Preferably, the groups present as substituents are chosen from:

carboxylic, in acid or salified form,
hydroxyl,
$C_1$-$C_4$ alkoxy,
($C_1$-$C_8$)alkoxycarbonyl,
thiol,
($C_1$-$C_4$)alkylthio,
amino,
($C_1$-$C_4$) mono- and di-alkylamino,
aminocarbonyl,
($C_1$-$C_2$) mono- and di-alkylaminocarbonyl,
($C_1$-$C_4$)alkylcarbonylamino,
phenyl, optionally substituted by one or more $C_1$-$C_2$ alkyl or hydroxyl.

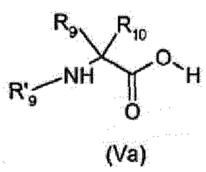

(Va)

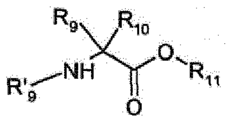

(Vb)

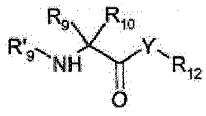

(Vc)

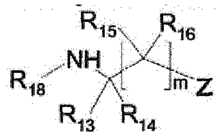

(Ve)

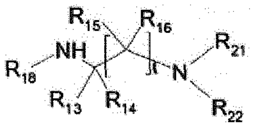

(Vf)

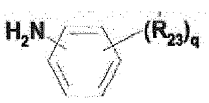

(Vg)

Preferably, the compounds of formula (Va) are chosen from arginine, asparagine, cysteine, glutamine, lysine, N-lauroyl lysine, methionine, phenylalanine, serine, threonine, and tyrosine. According to another variant, the compounds of formula (Va) are preferably: arginine, lysine or N-lauroyl lysine.

The non-heterocyclic nitrogen-based compound is preferably chosen from, lysine, N-lauroyl lysine, and arginine.

The amino compound may also be an amino polymer, in particular chosen from amino-based polymers containing primary amine groups. These are polymers resulting from the polymerization of monomers, the polymer comprising at least 2 primary amine groups $-NH_2$.

The amino polymer may especially be chosen from:

- poly(($C_2$-$C_5$)alkyleneimine)s, and in particular polyethyleneimines and polypropyleneimines, especially linear or branched poly(ethyleneimine)s (for example that sold under the reference 46,852-3 by Aldrich Chemical);
- poly(allylamine) (for example that sold under the reference 47,913-6 by Aldrich Chemical or by Beckmann-Kenko);
- polyvinylamines and copolymers thereof, in particular with vinylamides; mention may especially be made of vinylamine/vinylformamide copolymers (the vinylamine content possibly ranging from 30% to 90% by weight of the total weight of the polymer), such as those sold under the name Lupamin® 9030 by BASF;
- polyamino acids which have $NH_2$ groups, such as polylysine; for example that sold by JNC Corporation;
- aminodextran, such as that sold by CarboMer Inc;
- vinylamine/vinyl alcohol copolymers, such as those sold by Chemwill;
- acrylamidopropylamine polymers;
- chitosans, such as those sold under the names Zenvivo® Protect and Zenvivo Aqua by Clariant;
- dendrimers containing terminal $NH_2$ functions such as the PAMAM (polyamidoamines) dendrimers sold by Dendritech.

Preferably, the amino polymer is chosen from:

- poly(ethyleneimine)s;
- poly(allylamine);
- polylysine;
- vinylamine/vinylformamide copolymers;
- chitosans;
- dendrimers containing terminal $NH_2$ functions such as the PAMAM (polyamidoamines) dendrimers sold by Dendritech.

More preferentially, the amino polymer is a polylysine.
Advantageously, the amino polymer has a weight-average molecular weight ranging from 200 to 1 000 000 g/mol, preferably ranging from 300 to 500 000 g/mol.
Preferably, the amino polymer is water-soluble. The term "water-soluble polymer" means a polymer with a solubility in water at 25°C, of at least 0.1 g/l.
The nitrogen-based compound is preferably chosen from Nicotinamide Adenine Diphosphate, pyridoxine, adenosine triphosphate disodium salt, adenosine diphosphate, theobromine, adenosine, thymidine, guanine, cytidine, uridine, caffeine, xanthine, allantoin, nicotinic acid, trigonelline, paraxanthine, histidine, lysine, polylysine, N-Lauroyl lysine, arginine and adenine.
The nitrogen-based compound is preferentially chosen from pyridoxine, adenosine triphosphate disodium salt, adenosine, caffeine, allantoin, trigonelline, histidine, polylysine, N-Lauroyl lysine, arginine and adenine.
The nitrogen-based compound is more preferentially chosen from pyridoxine, adenosine triphosphate disodium salt, adenosine, allantoin, trigonelline, histidine, polylysine, arginine and adenine.
The nitrogen-based compound is more preferentially chosen from pyridoxine, adenosine triphosphate disodium salt, adenosine, allantoin, histidine and adenine.
The nitrogen-based compound is even more preferentially chosen from adenosine triphosphate disodium salt and histidine.
According to a preferred mode of the invention, the nitrogen-based compound is adenosine triphosphate disodium salt.
The nitrogen-based compound may be present in the coloured powder at a weight ratio of nitrogen-based compound/anthocyanin ranging from 1/10 to 10/1, preferably ranging from 1/3 to 3/1 by weight, preferentially ranging from 1/2 to 2/1 by weight.
Advantageously, when the coloured powder comprises an amino acid, it does not contain tannic acid or a gallotannin derivative thereof, or the mixtures thereof.
Tannic acid (or hydrolysable gallotannin) is a mixture of polygalloyl glucoses or of polygalloyl quinic acid esters with a number of galloyl units per molecule ranging from 2 to 12, depending on the plant source used to extract the tannic acid.
The chemical formula of commercial tannic acid commonly given is $C_{76}H_{52}O_{46}$, which corresponds to decagalloyl glucose. Gallotannin derivatives of tannic acid is intended to mean the polygalloyl glucose compounds and the polygalloyl quinic acid esters described previously which are present in tannic acid.
Commercial tannic acid is usually extracted from tara husk seeds (*Caesalpinia spinosa*), gall nuts or excrescences thereof which form on the young branches of *Quercus infectoria* and belonging to the species of *Quercus* L. (Fam. *Fagaceae*), or gall nuts of various species of sumac.
The coloured powder according to the invention is blue in colour.
Blue is the hue of the portion of the visible light spectrum located between green and indigo. One method for measuring colour, proposed by the Commission Internationale de l'Éclairage (CIE) (*International Commission on Illumination)*, is the CIE 1976 L*a*b* colour scale, hereinafter denoted CIELAB (CIE technical report, Colorimetry, 2nd Edition, CIE 15.2 - 1986, revised edition 1996). The CIELAB colour space is obtained by arranging the values L*, a*, b* as rectangular coordinates. The coordinates of an object and the light intensity are measured on a scale ranging from 0 (black) to 100 (absolute white).
The coordinates a* and b* do not have any specific numerical limits. The parameter a* ranges from pure green (negative a*) to pure red (positive a*), while the parameter b* ranges from pure blue (negative b*) to pure yellow (positive b*).
The hue angle $h_{ab}$ is calculated from the values a* and b* according to the equation:

$$h_{ab} = \mathrm{arctang}\,(b^*/a^*)$$

and for which $h_{ab}$ ranges from 0° to 90° if b* and a* are both positive, between 90° and 180° if b* is positive and a* is negative, between 180° and 270° if b* and a* are both negative, and between 270° and 360° if b* is negative and a* is

positive.

The colour blue within the context of the present invention means that the hue angle $h_{ab}$ is between 210° and 325°, and preferably between 250° and 300°.

Preparation process:

The coloured powder may be prepared in a first step by mixing in a buffered solution, having a pH of between 4.5 and 5.5, of the anthocyanin (especially in plant extract form), then adding the metal ions in the form of salts, and the nitrogen-based compound, and then the pH of the mixture is adjusted to between 4.5 and 6 (to cause the powder formed to precipitate).

In a second step, the solid phase of the mixture obtained in the first step is isolated, especially by centrifugation or by filtration, then dried, for example by lyophilization, to thereby recover the coloured powder.

The composition according to the invention comprising the coloured powder also comprises a physiologically acceptable medium, that is to say a medium compatible with the keratin fibres and/or materials of a living creature, in particular of humans, such as, for example, in a non-limiting manner, the skin, the lips, the nails, the hair, the eyelashes or the eyebrows.

The composition according to the invention may also contain cosmetic additives such as fragrances, preservatives, film-forming polymers, fillers, UV screening agents, thickeners, oils, gums, waxes, surfactants, moisturizers, vitamins, proteins and hydrolysates thereof, ceramides, antioxidants, anti-free-radical agents, organic solvents, inorganic pigments, organic pigments, nacres, dyes, water.

The amounts of these various additives are those conventionally used in the cosmetics field, and may range, for example, from 0.01% to 30% of the total weight of the composition. In general, the amounts are adjusted as a function of the formulation prepared.

The composition may be anhydrous or contain water.

A cosmetic composition according to the invention may be in the form of a product for making up the hair (in particular a coloured coating for the hair), the eyelashes, the eyebrows, the skin, the lips or the nails.

Other characteristics and advantages of the invention will emerge more clearly from the examples that follow, which are given as non-limiting illustrations. In the text hereinbelow or hereinabove, the proportions are given as weight percentages, unless otherwise indicated.

**Examples 1 to 11: Preparation of powders and colorimetric evaluation**

The following 3 solutions were first prepared:

Anthocyanin solution 1: aqueous-alcoholic extract of bilberry fruit (BILBERRY FRUIT PE25% ANTHOCYANOSIDES from NATUREX): 10 g (i.e. 2.5 g of anthocyanin active material in 1 litre of mixture comprising 90% by volume of 0.2 M sodium acetate buffer pH 5 in water and 10% by volume of ethanol.

Solution of amino compound 2: 0.2 M adenosine triphosphate (ATP) disodium salt in 0.1 M sodium acetate buffer
For the other nitrogen-based compounds tested, the solution 2 was prepared at 0.05 M or 0.1 M depending on the solubility so as to obtain a solution of the dissolved compound.

Solution of metal ions 3: Ammonium aluminium sulfate dodecahydrate at 0.2 M in 0.1 M sodium acetate buffer

Powder preparation method:

750 ml of solution 1 containing 10 g of bilberry extract (i.e. 2.5 g AM of anthocyanins) was mixed with 125 ml of solution 2 of ATP.

The mixture was stirred for 15 minutes at 25 °C, then 125 ml of solution 3 of ammonium aluminium sulfate dodecahydrate were added, then left again with stirring for 15 minutes under the same conditions; the pH of the mixture was then adjusted to pH 5.09 by addition of sodium hydroxide.

Thus, the amounts used in the final reaction mixture are as follows:

Bilberry extract: 5 g/l
ATP: 0.025 M
Ammonium aluminium sulfate dodecahydrate: 0.025 M

The mixture was then centrifuged to recover the final solid phase which was washed with water. This solid

phase was then frozen at -22°C then lyophilized, or else was dried in an oven under vacuum at 45°C.
A blue coloured powder was thus recovered.

Other powders were prepared by replacing the ATP with another nitrogen-based compound as indicated in the table below.

Evaluation of the colorimetric properties of the powders:

For each powder obtained, the colorimetric properties were evaluated according to the following protocol: A paste with the following composition was used (% by weight)

| | |
|---|---|
| - Polybutene (Indopol H 100 from Ineos) | 13.25% |
| - Polybutene (Indopol H 1500 from Ineos) | 10% |
| - Polyethylene wax (Performalene 500-L polyethylene from New Phase Technologies) | 6% |
| - hydrogenated myristic esters of olive oil fatty acids (PHYTOWAX OLIVE 14L48 from Sophim) | 4.5% |
| - Shea butter | 2.8% |
| - Caprylic, capric, isostearic, stearic, hydrostearic and adipic glyceryl fatty acid ester (SOFTISAN 649 from Cremer Oleo) | 7.6% |
| - Petroleum jelly (ULTIMA WHITE PET USP from Calumet Speciality) | 15% |
| - Diisostearyl malate | 17.5% |
| - 2-Octyldodecanol | 20% |
| - Silica Silylate (Aerogel VM-2270 from Dow Corning) | 0.8% |
| - Polyurethane powder (PLASTIC POWDER D 400 from TOSHIKI PIGMENT) | 2% |
| - Tocopherol acetate | 0.5% |
| - Dibutylpentaerythrityl tetrahydroxycinnamate | 0.05% |

A first paste was prepared by mixing 5% by weight of the powder to be evaluated and 95% by weight of the paste described above. Mixing was first carried out manually with a spatula, then continued in a mill to obtain a homogeneous paste.

The paste was then spread on an Erichsen contrast card, ref Typ 24/5, 50 μm thick; the colour of the deposit obtained spread on the white part and on the black part of the contrast card was measured at 3 points for each part of the card on the Minolta CM2600d colorimeter. The mean of 3 measurements made on each part of the card was calculated. Thus, the values $L_1$, $a_1$, $b_1$ for the deposit on the white part and $L_{1'}$, $a_{1'}$, $b_{1'}$ for the deposit on the black part were obtained.

The following parameters were taken (mean of the 3 measurements taken) from the colorimetric reading on the white part of the contrast card:

Lightness $L_1$

$$\text{Chromaticity } C = \left( (a_1)^2 + (b_1)^2 \right)^{1/2}$$

$$\text{Hue angle } H = \text{arctang } (b_1 / a_1)$$

Coverage was determined by the Contrast Ratio $CR = (Y'_1/Y_1) \times 100$

Y being the component of the Xyz colour space also measured by the colorimeter and corresponding to the percentage reflection.

The higher the CR value, the greater the coverage.

A second paste was prepared containing 10% by weight of the powder to be evaluated and 90% by weight of the paste described above. In this paste, the powder gives what is referred to as a full tone.

A third paste was prepared, containing 10% by weight of powder, 7.5% by weight of alumina/silica/trimethylolpropane-treated rutile titanium dioxide (TIPAQUE PF-671 COSMETIC GRADE from ISHIHARA SANGYO) and 82.5% by weight of the paste described above. In this paste, the powder is mixed with the white titanium dioxide pigment, thereby giving what is referred to as a reduced tone of the powder in this paste.

The tinting strength (TS) of the powder was then determined according to the following protocol:

2 g of the second paste were deposited in a small cup (made of galvanized steel and rectangular in shape; length = 25.8 mm, width = 22.8 mm, height = 3.6 mm), then the same operation was carried out with the third paste.

The content of each small cup was read with the colorimeter to measure the colorimetric parameters $L_2$, $a_2$, $b_2$ of the second paste (full tone) and those of the third paste ($L_3$, $a_3$, $b_3$) (reduced tone).

$$\text{The tinting strength TS of the powder} = [ (L_3 - L_2)^2 + (a_3 - a_2)^2 + (b_3 - b_2)^2 ]^{1/2}$$

The smaller the TS value, the greater the tinting strength.

The photostability of the powder was determined according to the following protocol:
The small cup containing the third paste (reduced tone) was exposed for one hour to irradiation under a LOT Oriel solar simulator (1600 W power). Then, in the body of the irradiated paste, the parameters $L_{3'}$, $a_{3'}$, $b_{3'}$ were measured with the colorimeter.

The photostability was determined by calculating the difference ΔE between the colour measurements taken before and after irradiation:

$$\text{Photostability} = \Delta E = ((L_{3'}-L_3)^2 + (a_{3'}-a_3)^2 + (b_{3'}-b_3)^2)^{½}$$

The following results were obtained:

| Example powder | Nitrogen-based compound | C | ΔE | TS | CR (%) | L1 | H |
|---|---|---|---|---|---|---|---|
| 1 | Adenosine triphosphate disodium salt | 22.9 | 3.4 | 22.2 | 51.2 | 35.8 | 277.1 |
| 2 | Histidine | 12.1 | 6.7 | 15.9 | 80.6 | 26.8 | 286.2 |
| 3 | Adenine | 17.0 | 9.5 | 21.0 | 51.4 | 35.8 | 255.3 |
| 4 | Allantoin | 15.4 | 8.3 | 18.8 | 72.0 | 29.6 | 285.6 |
| 5 | Adenosine | 14.5 | 8.3 | 20.3 | 64.1 | 32.4 | 273.0 |
| 6 | Pyridoxine | 13.3 | 8.3 | 19.8 | 67.9 | 31.1 | 274.9 |
| 7 | trigonelline | 17.1 | 10.2 | 19.8 | 44.4 | 36.7 | 277.6 |
| 8 | Arginine | 15.4 | 11.1 | 18.9 | 49.4 | 34.6 | 273.1 |
| 9 | Polylysine (1) | 12.6 | 13.2 | 20.5 | 49.0 | 37.5 | 273.7 |
| 10 | Lauroyl lysine(2) | 14.4 | 16.7 | 21.0 | 42.7 | 38.0 | 268.2 |
| 11 | Caffeine | 17.7 | 16.9 | 22.2 | 47.2 | 37.3 | 264.4 |
| (1) polylysine sold under the reference P8920 by Aldrich<br>(2) sold under the reference AMIHOPELL by Ajinomoto | | | | | | | |

The results obtained show that:
All the powders are blue in colour (hue angle between 264 and 287).

The powders have good properties of chromaticity and photostability and also at least one good property from properties of coverage, tinting strength and lightness.

**Comparative Example 12:**

A powder 1' was prepared, similar to the powder 1 described above but with modification of the concentrations of the ingredients used during the preparation, as described below:

Bilberry extract: 8.8 g/l
ATP: 11.2 g/l (i.e. 0.02 M)
Ammonium aluminium sulfate dodecahydrate: 19 g/l (i.e. 0.0419 M)

A powder 1'a, similar to the powder 1' but without using ATP (obtained with the bilberry extract and ammonium

aluminium sulfate dodecahydrate) and a powder 1'b, without using ammonium aluminium sulfate dodecahydrate (obtained with the bilberry extract and ATP), were also prepared.

The colorimetric properties of these powders were measured, and the yield of powder obtained was also noted.

The following results were obtained:

| Example powder | Yield (%) | C | ΔE | a | TS | CR (%) | H |
|---|---|---|---|---|---|---|---|
| **1'** | 35.90 | 23.1 | 2.9 | 5.1 | 20.3 | 59.6 | 283 |
| **1'a** | 40 | 18.6 | 7.6 | 6.5 | 17.3 | 60.7 | 291 |
| **1'b** | 2.1 | 8.8 | - | -0.8 | - | 32.9 | 265 |

The powder 1' (ATP) has good chromaticity, good coverage and a good hue angle, and also good photostability.

The powder 1'a has good tinting strength, a good hue angle and good coverage, but poorer chromaticity and too red a colour (a = 6.5).

The powder 1'b was obtained with a very low yield and has very poor chromaticity, and also a poorer hue angle and coverage.

The colorimetric properties of the powder 1' according to the invention were also compared with those of another powder described in the prior art (example 3 from FR2994693) prepared from bilberry extract, iron sulfate and tannic acid.

The following results were obtained:

| Example powder | C | a | TS | CR (%) | L | H |
|---|---|---|---|---|---|---|
| **1' (ATP)** | 23.1 | 5.1 | 20.3 | 59.6 | 32.1 | 283 |
| **Ex. 3 from** FR2994693 | 5.93 | 1.88 | 14.1 | 62.5 | 31 | 288 |

The results obtained show that the powder of example 3 of FR2994693 based on bilberry/iron sulfate/tannic acid has poor chromaticity and hence an insufficiently vibrant blue colour.

## Examples 13 and 14:

2 powders according to the invention were prepared with different salts using the following final concentrations according to the procedure described above in examples 1 to 11:

Bilberry extract: 8.8 g/l
ATP: 11.2 g (i.e. 0.02 M)
salt: $AlNH_4(SO_4)_2$ dodecahydrate at 0.0419 M (example 1') or aluminium chloride at 0.055 M (example 13) or zinc sulfate at 0.052 M (example 16)

The following results were obtained:

| Example powder | salt | Yield (%) | C | TS | CR (%) | L | a | H |
|---|---|---|---|---|---|---|---|---|
| **1'** | $AlNH_4(SO_4)_2$ dodecahydrate | 35.9 | 23.1 | 20.3 | 59.6 | 32.1 | 5.1 | 283 |
| **13** | Aluminium chloride | 71.8 | 24.6 | 22.54 | 50.1 | 34.3 | 4.1 | 279 |
| **14** | Zinc sulfate | 21.8 | 26.5 | 20.88 | 69.5 | 28.9 | 10.3 | 293 |

The powder 14 (zinc sulfate) is the best performer: it has good hue angle, chromaticity, tinting strength and coverage properties. Nonetheless, it has a redder "a".

The powder 13 (aluminium chloride) has good hue angle, chromaticity, and lightness properties. The yield is higher than that of powder 14.

## Examples 15 and 16:

2 powders according to the invention were prepared with black rice extracts using the following final concentrations according to the procedure described above in examples 1 to 11:

Black rice extract: 8.8 g/l
ATP: 11.2 g/l (i.e. 0.02 M)
Ammonium aluminium sulfate dodecahydrate: 19 g/l (i.e. 0.0419 M)

For the powder 15, the black rice extract used is that sold under the name RED RICE RED by Chenguang Biotech group.

For the powder 16, the black rice extract used is that sold under the name BLACK RICE extract by DAXINGANLING LINGONBERRY GROUP.

The colorimetric properties of these powders were measured.

The following results were obtained:

| Example pigment | Extracts | C | ΔE | TS | CR (%) | L | H |
|---|---|---|---|---|---|---|---|
| **1'** | Bilberry | 23.1 | 2.9 | 20.3 | 59.6 | 32.1 | 283 |
| **15** | Black rice | 18.1 | 3.7 | 18.2 | 55.9 | 32.2 | 278 |
| **16** | Black rice | 19.7 | 3.7 | 19.1 | 57.2 | 32.1 | 281 |

The powders 15 and 16 have good chromaticity and photostability properties.

**Example 17: eyeshadow example**

The following eyeshadow composition is prepared:

| | |
|---|---|
| Magnesium stearate | 4% |
| Talc | 30% |
| Glyceryl triisostearate | 5% |
| Hydrogenated polydecene | 5% |
| Blue powder according to one of examples 1 to 11 or 1' or 13 to 16 q.s. | 100% |

The shadow applied to the eyelids has a very attractive blue colour.

## Claims

1. Coloured, water-insoluble powder comprising the combination of an anthocyanin, a complexed metal chosen from aluminium or zinc or the mixture thereof, and a nitrogen-based compound chosen from:

(i) heterocyclic nitrogen-based compounds of formula (IVa):

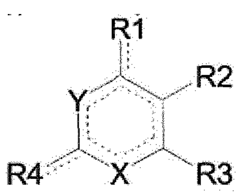
(IVa)

in which:

the --- bonds may signify a single bond or a double bond, it being understood that 2 contiguous bonds cannot be double bonds, the nitrogen-based heterocycles being able to be saturated, unsaturated or aromatic;
X denotes -C(Rs)= or -N= or $-N(R_6)-$ ;
Y denotes -N= or $-N(R_7)-$ or $-(R_8)N^+-$, $An^-$

$R_1$ denotes an amino radical, a (C1-C4)alkylamino radical, a (C1-C4)dialkylamino radical, an oxygen atom (in carbonyl form), a hydrogen atom, or a C1-C4 alkyl radical, optionally substituted by at least one hydroxyl group, such as methyl;
$R_2$ denotes a hydrogen atom, a hydroxyl radical, a carboxyl ($-CO_2H$) radical, or a C1-C4 alkyl radical such as methyl;
$R_3$ denotes a hydrogen atom or a C1-C4 alkyl radical optionally substituted by one or more hydroxyl radicals, such as hydroxymethyl;
$R_4$ denotes a hydrogen atom, an amino radical or an oxygen atom (in carbonyl form);
$R_5$ denotes a hydrogen atom or a C1-C4 alkyl radical optionally substituted by one or more hydroxyl radicals, such as hydroxymethyl;
$R_6$ denotes a hydrogen atom, a C1-C4 alkyl radical such as methyl, or a radical of formula (aa):

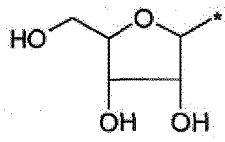
(aa)

$R_7$ denotes a hydrogen atom or a C1-C4 alkyl radical such as methyl;
$R_8$ denotes a C1-C4 alkyl radical such as methyl;
$R_2$ and $R_3$ possibly forming, together with the carbon atoms which bear them, a saturated or unsaturated 4- to 8-membered (hetero)cycle, 1 or more non-adjacent ring members of which may denote a nitrogen atom -N= or an $-N(R_9)-$ radical, with Rg denoting a hydrogen atom or a radical $R_{10}$ of formula:

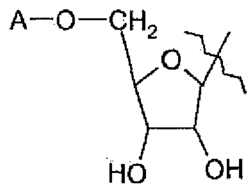
($R_{10}$)

in which A is chosen from a hydrogen atom, or a (dd), (ee) or (ff) radical as follows:

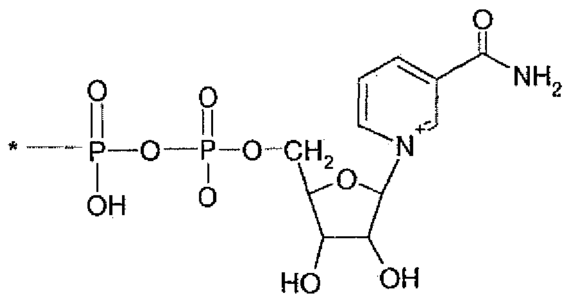
(dd)

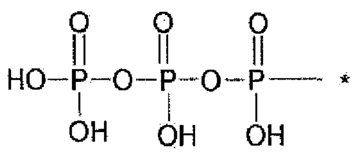
(ee)

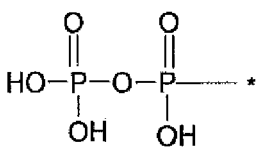
(ff)

* indicates the point of attachment of the radical A to the oxygen atom of R10
An⁻ denotes a cosmetically acceptable anion, and the salts thereof

(ii) heterocyclic nitrogen-based compounds of formula (IVb):

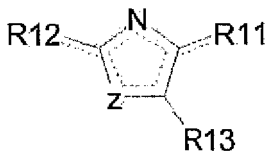

(IVb)

in which:

the --- bonds may signify a single bond or a double bond, it being understood that 2 contiguous bonds cannot be double bonds, the nitrogen-based heterocycles being able to be saturated, unsaturated or aromatic;
$R_{11}$ denotes an oxygen atom (in carbonyl form) or a $-CH_2-CH(NH_2)-CO_2H$ radical or a -COOH group;
$R_{12}$ denotes a hydrogen atom or an oxygen atom (in carbonyl form);
$R_{13}$ denotes a hydrogen atom or an $-NH-CO-NH_2$ radical;
Z denotes a nitrogen atom -N= or an-$N(R_{14})$- radical, in which $R_{14}$ denotes a hydrogen atom or a $-CH_2-CH(NH_2)-CO_2H$ radical, or a -CH2- radical; and the salts thereof

(iii) nitrogen-based compounds of formula (Va):

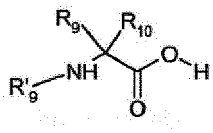

(Va)

in which:

- Rg represents a hydrogen atom or a linear or branched $C_1$-$C_6$ alkyl radical, optionally substituted by one or more identical or different groups chosen from hydroxyl, hydroxycarbonyl, thiol, ($C_1$-$C_4$)alkylthio, amido, amino or guanidine radicals, or a phenyl radical, optionally substituted by one or more hydroxyls;
- R'g represents a hydrogen, a $C_1$-$C_4$ alkyl radical, or an unsubstituted benzyl radical, or a $C_2$-$C_{16}$ acyl radical; R'g preferably represents a hydrogen atom;
- $R_{10}$ represents a hydrogen or a $C_1$-$C_4$ alkyl radical; $R_{10}$ preferably represents a hydrogen atom and the salts thereof

(iv) amino polymers
with the proviso that, when the nitrogen-based compound is an amino acid, then the coloured powder does not contain tannic acid or a gallotannin derivative thereof, or the mixtures thereof.

2. Powder according to the preceding claim, **characterized in that** the anthocyanin is a compound of formula (I):

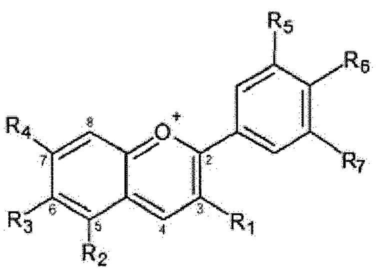

in which $R_1$, $R_2$ and $R_4$ independently denote H, OH, OMe, a sugar unit or an acylated sugar unit, the acyl group of which contains 2 to 15 carbon atoms, it being understood that at least one of the radicals $R_1$, $R_2$ and $R_4$ denotes a sugar unit or an acylated sugar unit, and $R_3$, $R_5$, $R_6$ and $R_7$ independently denote H, OH or OMe.

**3.** Powder according to either one of the preceding claims, **characterized in that** the anthocyanin is derived from plant material chosen from black carrot, elderberry, hibiscus, purple corn, black potato, red cabbage, red onion, purple potato, black grape, cranberry, strawberry, raspberry, aronia, black soybean, blackcurrant, radish, gooseberry, blueberry, cherry, aubergine and black rice.

**4.** Powder according to any one of the preceding claims, **characterized in that** the anthocyanin is present in the coloured powder in a content ranging from 0.05% to 50% by weight, relative to the total weight of dry matter of the coloured powder, preferably ranging from 0.3% to 30% by weight, preferentially ranging from 5% to 30% by weight, and more preferentially ranging from 10% to 30% by weight.

**5.** Powder according to any one of the preceding claims, **characterized in that** the complexed metal is aluminium,

**6.** Powder according to any one of Claims 1 to 4, **characterized in that** the complexed metal is derived from salts chosen from aluminium sulfate, ammonium aluminium sulfate, aluminium acetate, aluminium chloride, aluminium chlorohydrate, zinc acetate, zinc gluconate, zinc chloride, zinc sulfate; and mixtures thereof, and preferably chosen from ammonium aluminium sulfate, aluminium chloride, zinc sulfate, and mixtures thereof.

**7.** Powder according to any one of the preceding claims, **characterized in that** the complexed metal is present at a weight ratio of metal ions/anthocyanin ranging from 0.01/1 to 10/1, preferably ranging from 0.05/1 to 5/1.

**8.** Powder according to any one of the preceding claims, **characterized in that** the nitrogen-based compound is a compound of formula (IVa) or (IVb) and is chosen from nicotinamide adenine diphosphate, adenosine triphosphate disodium salt, pyridoxine, adenosine diphosphate, theobromine, adenosine, thymidine, guanine, cytidine, uridine, caffeine, xanthine, nicotinic acid, trigonelline, paraxanthine, adenine, histidine, allantoin, tryptophan and proline.

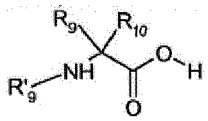

(Va)

**9.** Powder according to any one of Claims 1 to 7, **characterized in that** the nitrogen-based compound is a compound of formula (Va) and is chosen from, lysine, N-lauroyl lysine or arginine.

**10.** Powder according to any one of Claims 1 to 7, **characterized in that** the nitrogen-based compound is an amino polymer chosen from:

- poly(($C_2$-$C_5$)alkyleneimine)s;
- poly(allylamine);
- polyvinylamines and copolymers thereof, especially with vinylamides;
- aminodextran;
- vinylamine/vinyl alcohol copolymers;
- acrylamidopropylamine polymers;
- chitosans.

**11.** Powder according to any one of Claims 1 to 7 and 10, **characterized in that** the nitrogen-based compound is polylysine.

**12.** Powder according to any one of Claims 1 to 7, **characterized in that** the nitrogen-based compound is chosen from Nicotinamide Adenine Diphosphate, pyridoxine, adenosine triphosphate disodium salt, adenosine diphosphate, theobromine, adenosine, thymidine, guanine, cytidine, uridine, caffeine, xanthine, allantoin, nicotinic acid, trigonelline, paraxanthine, histidine, -lysine, polylysine, N-Lauroyl lysine, arginine, adenine; preferably chosen from pyridoxine, adenosine triphosphate disodium salt, adenosine, caffeine, allantoin, trigonelline, histidine, polylysine, N-Lauroyl lysine, arginine, adenine; more preferentially chosen from pyridoxine, adenosine triphosphate disodium salt, adenosine, allantoin, trigonelline, histidine, polylysine, arginine and adenine.

**13.** Powder according to any one of Claims 1 to 7, **characterized in that** the nitrogen-based compound is chosen from pyridoxine, adenosine triphosphate disodium salt, adenosine, allantoin, histidine and adenine.

**14.** Powder according to any one of Claims 1 to 7, **characterized in that** the nitrogen-based compound is chosen from adenosine triphosphate disodium salt and histidine.

**15.** Powder according to any one of Claims 1 to 7, **characterized in that** the nitrogen-based compound is adenosine triphosphate disodium salt.

**16.** Powder according to any one of Claims 1 to 7, **characterized in that** the nitrogen-based compound is present at a weight ratio of nitrogen-based compound/anthocyanin ranging from 1/10 to 10/1, preferably ranging from 1/3 to 311 by weight, preferentially ranging from 112 to 211 by weight.

**17.** Powder according to any one of the preceding claims, **characterized in that** it has a blue colour and a hue angle of between 210° and 325°, and preferably between 250° and 300°.

**18.** Cosmetic composition comprising, in a physiologically acceptable medium, a coloured powder according to any one of the preceding claims.

**19.** Cosmetic process for the treatment of keratin materials, comprising the application, to the keratin materials, of a coloured powder according to one of Claims 1 to 17 or of a composition according to Claim 18.

**20.** Process according to the preceding claim, **characterized in that** it is a process for making up keratin materials.

**Patentansprüche**

**1.** Gefärbtes wasserunlösliches Pulver, umfassend die Kombination eines Anthocyanins, eines komplexgebundenen Metalls ausgewählt aus Aluminium und Zink und dem Gemisch davon, und einer Verbindung auf Stickstoffbasis ausgewählt aus:

(i) heterocyclischen Verbindungen auf Stickstoffbasis der Formel (IVa):

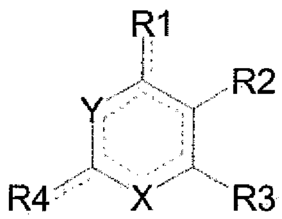

(IVa)

wobei:

die --- -Bindungen eine Einfachbindung oder eine Doppelbindung bedeuten können, wobei zu beachten ist, dass 2 benachbarte Bindungen nicht Doppelbindungen sein können, wobei die Heterocyclen auf Stickstoffbasis gesättigt, ungesättigt oder aromatisch sein können;
X -C(Rs)= oder -N= oder -N($R_6$)- darstellt;
Y -N= oder -N($R_7$)- oder -($R_8$)$N^+$-, $An^-$ darstellt;
$R_1$ einen Aminorest, einen (C1-C4)Alkylaminorest, einen (C1-C4)Dialkylaminorest, ein Sauerstoffatom (in Carbonylform), ein Wasserstoffatom oder einen C1-C4-Alkylrest, gegebenenfalls substituiert mit wenigstens einer Hydroxygruppe, wie z. B. Methyl, darstellt;
$R_2$ ein Wasserstoffatom, einen Hydroxyrest, einen Carboxyrest (-$CO_2$H) oder einen Cl-C4-Alkylrest, wie z. B. Methyl, darstellt;
$R_3$ ein Wasserstoffatom oder einen Cl-C4-Alkylrest, gegebenenfalls substituiert mit einem oder mehreren

Hydroxyresten, wie z. B. Hydroxymethyl, darstellt;

$R_4$ ein Wasserstoffatom, einen Aminorest oder ein Sauerstoffatom (in Carbonylform) darstellt;

$R_5$ ein Wasserstoffatom oder einen C1-C4-Alkylrest, gegebenenfalls substituiert mit einem oder mehreren Hydroxyresten, wie z. B. Hydroxymethyl, darstellt;

$R_6$ ein Wasserstoffatom, einen C1-C4-Alkylrest, wie z. B. Methyl, oder einen Rest der Formel (aa) darstellt:

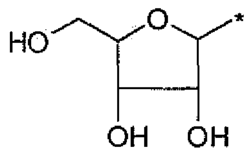

(aa)

$R_7$ ein Wasserstoffatom oder einen C1-C4-Alkylrest, wie z. B. Methyl, darstellt;

$R_8$ einen C1-C4-Alkylrest, wie z. B. Methyl, darstellt;

$R_2$ und $R_3$ gegebenenfalls zusammen mit den Kohlenstoffatomen, die sie tragen, einen gesättigten oder ungesättigten, 4- bis 8-gliedrigen (Hetero)cyclus bilden, bei dem 1 oder mehr nichtbenachbarte Ringelemente ein Stickstoffatom -N= oder einen -N($R_9$)-Rest darstellen können, wobei $R_9$ ein Wasserstoffatom oder einen Rest $R_{10}$ der Formel:

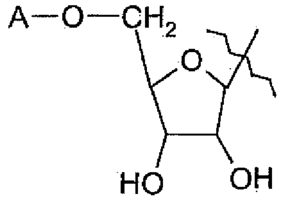

**($R_{10}$)**

darstellt, wobei A ausgewählt ist aus einem Wasserstoffatom und einem (dd)-, (ee) - oder (ff)-Rest, wie folgt:

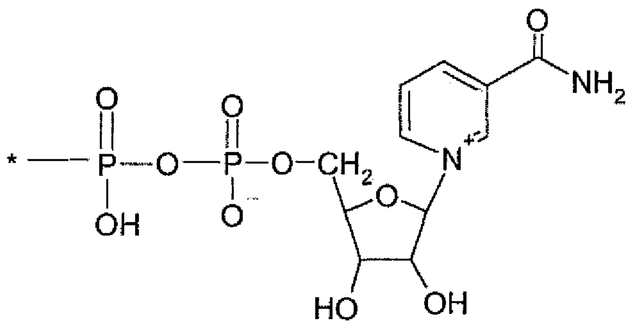

(dd)

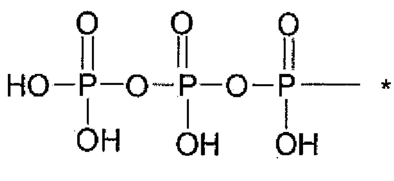

(ee)

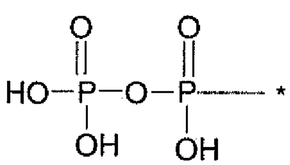

(ff)

wobei * den Punkt der Befestigung des Rests A an dem Sauerstoffatom von R10 zeigt,

An⁻ ein kosmetisch annehmbares Anion darstellt,

und den Salzen davon,

(ii) heterocyclischen Verbindungen auf Stickstoffbasis der Formel (IVb):

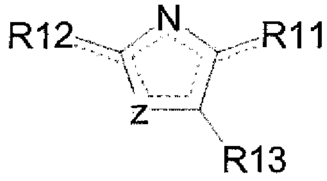

(IVb)

wobei:

die --- -Bindungen eine Einfachbindung oder eine Doppelbindung bedeuten können, wobei zu beachten ist, dass 2 benachbarte Bindungen nicht Doppelbindungen sein können, wobei die Heterocyclen auf Stickstoffbasis gesättigt, ungesättigt oder aromatisch sein können,
$R_{11}$ ein Sauerstoffatom (in Carbonylform) oder einen -$CH_2$-CH($NH_2$)-$CO_2$H-Rest oder eine -COOH-Gruppe darstellt;
$R_{12}$ ein Wasserstoffatom oder ein Sauerstoffatom (in Carbonylform) darstellt;
$R_{13}$ ein Wasserstoffatom oder einen -NH-CO-$NH_2$-Rest darstellt;
Z ein Stickstoffatom -N= oder einen -N($R_{14}$)-Rest darstellt, wobei $R_{14}$ ein Wasserstoffatom oder einen -$CH_2$-CH($NH_2$)-COsH-Rest oder einen -CH2-Rest darstellt;
und den Salzen davon,

(iii) Verbindungen auf Stickstoffbasis der Formel (Va);

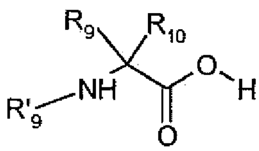

(Va)

wobei:

- $R_9$ ein Wasserstoffatom oder einen linearen oder verzweigten $C_1$-$C_6$-Alkylrest, gegebenenfalls substituiert mit einem oder mehreren gleichen oder verschiedenen Gruppen ausgewählt aus Hydroxy-, Hydroxycarbonyl-, Thiol-, ($C_1$-$C_4$)Alkylthio-, Amido-, Amino- und Guanidinresten, oder einen Phenylrest, gegebenenfalls substituiert mit einem oder mehreren Hydroxy, darstellt;
- $R'_9$ einen Wasserstoff, einen $C_1$-$C_4$-Alkylrest oder einen unsubstituierten Benzylrest oder einen $C_2$-$C_{16}$-Acylrest darstellt; wobei $R'_9$ vorzugsweise ein Wasserstoffatom darstellt;
- $R_{10}$ einen Wasserstoff oder einen $C_1$-$C_4$-Alkylrest darstellt; wobei $R_{10}$ vorzugsweise ein Wasserstoffatom darstellt;

und den Salzen davon,
(iv) Aminopolymeren,
mit der Maßgabe, dass, wenn die Verbindung auf Stickstoffbasis eine Aminosäure ist, das gefärbte Pulver kein (e) Tanninsäure oder Gallotanninderivat davon oder die Gemische davon enthält.

2. Pulver gemäß dem vorstehenden Anspruch, **dadurch gekennzeichnet, dass** das Anthocyanin eine Verbindung der Formel (I) ist:

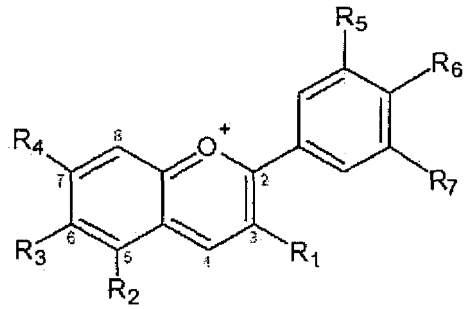

wobei $R_1$, $R_2$ und $R_4$ unabhängig H, OH, OMe, eine Zuckereinheit oder eine acylierte Zuckereinheit, deren Acylgruppe 2 bis 15 Kohlenstoffatome enthält, darstellen, mit der Maßgabe, dass wenigstens einer der Reste $R_1$, $R_2$ und $R_4$ eine Zuckereinheit oder eine acylierte Zuckereinheit darstellt und $R_3$, $R_5$, $R_6$ und $R_7$ unabhängig H, OH oder OMe darstellen.

3. Pulver gemäß einem der vorstehenden Ansprüche, **dadurch gekennzeichnet, dass** das Anthocyanin von einem Pflanzenmaterial ausgewählt aus schwarzer Karotte, Holunder, Hibiskus, Purpurmais, schwarzer Kartoffel, Rotkohl, roter Zwiebel, Purpurkartoffel, schwarzen Trauben, Cranberry, Erdbeere, Himbeere, Aronia, schwarzer Sojabohne, schwarzer Johannisbeere, Rettich, Stachelbeere, Heidelbeere, Kirsche, Aubergine und schwarzem Reis abgeleitet ist.

4. Pulver gemäß einem der vorstehenden Ansprüche, **dadurch gekennzeichnet, dass** das Anthocyanin in dem gefärbten Pulver in einem Gehalt in dem Bereich von 0,05 Gew.-% bis 50 Gew.-%, bezogen auf das Gesamtgewicht an Trockenmaterial des gefärbten Pulvers, vorhanden ist, vorzugsweise in dem Bereich von 0,3 Gew.-% bis 30 Gew.-%, vorzugsweise in dem Bereich von 5 Gew.-% bis 30 Gew.-% und bevorzugter in dem Bereich von 10 Gew.-%bis 30 Gew.-%.

5. Pulver gemäß einem der vorstehenden Ansprüche, **dadurch gekennzeichnet, dass** das komplexgebundene Metall Aluminium ist.

6. Pulver gemäß einem der Ansprüche 1 bis 4, **dadurch gekennzeichnet, dass** das komplexgebundene Metall abgeleitet ist von Salzen ausgewählt aus Aluminiumsulfat, Ammoniumaluminiumsulfat, Aluminiumacetat, Aluminiumchlorid, Aluminiumchlorhydrat, Zinkacetat, Zinkgluconat, Zinkchlorid, Zinksulfat und Gemischen davon und vorzugsweise ausgewählt ist aus Ammoniumaluminiumsulfat, Aluminiumchlorid, Zinksulfat und Gemischen davon.

7. Pulver gemäß einem der vorstehenden Ansprüche, **dadurch gekennzeichnet, dass** das komplexgebundene Metall in einem Gewichtsverhältnis von Metallionen/Anthocyanin im Bereich von 0,01/1 bis 10/1, vorzugsweise in dem Bereich von 0,05/1 bis 5/1, vorhanden ist.

8. Pulver gemäß einem der vorstehenden Ansprüche, **dadurch gekennzeichnet, dass** die Verbindung auf Stickstoffbasis eine Verbindung der Formel (IVa) oder (IVb) ist und ausgewählt ist aus Nicotinamidadenindiphosphat, Adenosintriphosphat-Dinatriumsalz, Pyridoxin, Adenosindiphosphat, Theobromin, Adenosin, Thymidin, Guanin, Cytidin, Uridin, Coffein, Xanthin, Nicotinsäure, Trigonellin, Paraxanthin, Adenin, Histidin, Allantoin, Tryptophan und Prolin,

9. Pulver gemäß einem der Ansprüche 1 bis 7, **dadurch gekennzeichnet, dass** die Verbindung auf Stickstoffbasis der Formel (Va) ausgewählt ist aus Guanidin (insbesondere in Hydrochloridform), Lysin, N-Lauroyllysin und Arginin.

10. Pulver gemäß einem der Ansprüche 1 bis 7, **dadurch gekennzeichnet, dass** die Verbindung auf Stickstoffbasis ein Aminpolymer ist, ausgewählt aus:

- Poly(($C_2$-$C_5$) alkylenimin) en;
- Poly (allylamin) ;
- Polyvinylaminen und Copolymeren davon, insbesondere mit Vinylamiden;
- Aminodextran;
- Vinylamin/Vinylalkohol-Copolymeren;
- Acrylamidopropylaminpoylmeren;
- Chitosanen.

11. Pulver gemäß einem der Ansprüche 1 bis 7 und 10, **dadurch gekennzeichnet, dass** die Verbindung auf Stickstoffbasis Polylysin ist.

12. Pulver gemäß einem der Ansprüche 1 bis 7, **dadurch gekennzeichnet, dass** die Verbindung auf Stickstoffbasis ausgewählt ist aus Nicotinamidadenindiphosphat, Pyridoxin, Adenosintriphosphat-Dinatriumsalz, Adenosindiphosphat, Theobromin, Adenosin, Thymidin, Guanin, Cytidin, Uridin, Coffein, Xanthin, Allantoin, Nicotinsäure, Trigonellin, Paraxanthin, Histidin, Guanidin (insbesondere in Hydrochloridform), Lysin, Polylysin, N-Lauroyllysin,Arginin, Adenin; vorzugsweise ausgewählt aus Pyridoxin, Adenosintriphosphat-Dinatriumsalz, Adenosin, Coffein, Allantoin, Trigonellin, Histidin, Polylysin, N-Lauroyllysin, Arginin, Adenin; weiter bevorzugt ausgewählt ist aus Pyridoxin, Adenosintriphosphat-Dinatriumsalz, Adenosin, Allantoin, Trigonellin, Histidin, Polylysin, Arginin und Adenin.

13. Pulver gemäß einem der Ansprüche 1 bis 7, **dadurch gekennzeichnet, dass** die Verbindung auf Stickstoffbasis ausgewählt ist aus Pyridoxin, Adenosintriphosphat-Dinatriumsalz, Adenosin, Allantoin, Histidin und Adenin.

14. Pulver gemäß einem der Ansprüche 1 bis 7, **dadurch gekennzeichnet, dass** die Verbindung auf Stickstoffbasis ausgewählt ist aus Adenosintriphosphat-Dinatriumsalz und Histidin.

15. Pulver gemäß einem der Ansprüche 1 bis 7, **dadurch gekennzeichnet, dass** die Verbindung auf Stickstoffbasis Adenosintriphosphat-Dinatriumsalz ist.

16. Pulver gemäß einem der Ansprüche 1 bis 7, **dadurch gekennzeichnet, dass** die Verbindung auf Stickstoffbasis in einem Gewichtsverhältnis von Verbindung auf Stickstoffbasis/Anthocyahin im Bereich von 1/10 bis 10/1, vorzugsweise im Bereich von 1/3 bis 3/1, vorzugsweise im Bereich von 1/2 bis 2/1, nach Gewicht vorhanden ist.

17. Pulver gemäß einem der vorstehenden Ansprüche, **dadurch gekennzeichnet, dass** es eine blaue Farbe und einen Farbtonwinkel zwischen 210° und 325°, vorzugsweise zwischen 250° und 300°, aufweist.

18. Kosmetische Zusammensetzung, umfassend ein gefärbtes Pulver gemäß einem der vorstehenden Ansprüche in einem physiologisch annehmbaren Medium.

19. Kosmetisches Verfahren zur Behandlung von Keratinmaterialien, umfassend das Aufbringen eines gefärbten Pulvers gemäß einem der Ansprüche 1 bis 17 oder einer Zusammensetzung gemäß Anspruch 18 auf die Keratinmaterialien.

20. Verfahren gemäß dem vorstehenden Anspruch, **dadurch gekennzeichnet, dass** es ein Verfahren zum Make-up von Keratinmaterialien ist.

**Revendications**

1. Poudre colorée insoluble dans l'eau comprenant la combinaison d'une anthocyanine, d'un métal complexé choisi parmi l'aluminium, le zinc ou le mélange correspondant, et d'un composé azoté choisi parmi :

(i) des composés azotés hétérocycliques de formule (IVa) :

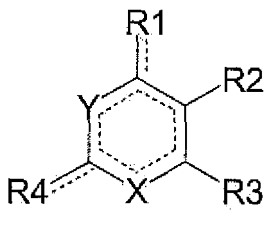

(IVa)

dans laquelle :

les liaisons - - - peuvent signifier une simple liaison ou une double liaison, étant entendu que 2 liaisons contigües ne peuvent pas être des doubles liaisons, les hétérocycles azotés pouvant être saturés, insaturés ou aromatiques ;

X désigne -C($R_5$)= ou -N= ou -N($R_6$)- ;
* désigne -N= ou -N($R_7$)- ou -($R_8$)$N^+$-, $An^-$
$R_1$ désigne un radical amino, un radical (Cl-C4)alkylamino, un radical (C1-C4)dialkylamino, un atome d'oxygène (sous forme de carbonyle), un atome d'hydrogène, ou un radical Cl-C4-alkyle, éventuellement substitué par au moins un groupe hydroxyle, tel que méthyle ;
$R_2$ désigne un atome d'hydrogène, un radical hydroxyle, un radical carboxyle (-$CO_2$H), ou un radical C1-C4-alkyle tel que méthyle ;
$R_3$ désigne un atome d'hydrogène ou un radical Cl-C4-alkyle éventuellement substitué par un ou plusieurs radicaux hydroxyle, tel qu'hydroxyméthyle ;
$R_4$ désigne un atome d'hydrogène, un radical amino ou un atome d'oxygène (sous forme de carbonyle) ;
$R_5$ désigne un atome d'hydrogène ou un radical Cl-C4-alkyle éventuellement substitué par un ou plusieurs radicaux hydroxyle, tel qu'hydroxyméthyle ;
$R_6$ désigne un atome d'hydrogène, un radical Cl-C4-alkyle tel que méthyle, ou un radical de formule (aa) :

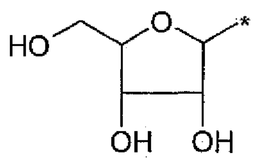
(aa)

$R_7$ désigne un atome d'hydrogène ou un radical C1-C4-alkyle tel que méthyle ;
$R_8$ désigne un radical Cl-C4-alkyle tel que méthyle ;
$R_2$ et $R_3$ pouvant former, conjointement avec les atomes de carbone qui les portent, un (hétéro) cycle saturé ou insaturé à 4 à 8 chaînons, dont 1 ou plusieurs éléments de cycle non adjacents peuvent désigner un atome d'azote -N= ou un radical -N($R_9$) -, $R_9$ désignant un atome d'hydrogène ou un radical $R_{10}$ de formule :

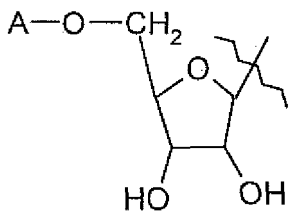
($R_{10}$)

dans laquelle A est choisi parmi un atome d'hydrogène, et un radical (dd), (ee) ou (ff) comme suit :

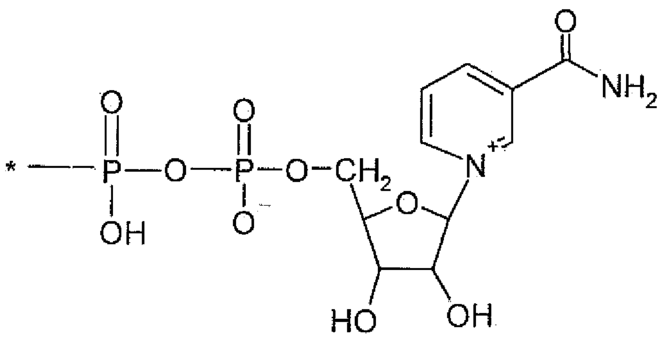
(dd)

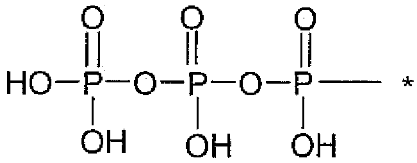
(ee).

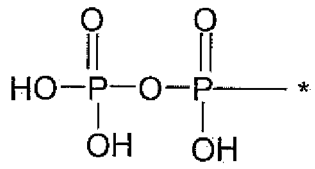

(ff)

* indique le point de fixation du radical A à l'atome d'oxygène de R10
An⁻ désigne un anion acceptable sur le plan cosmétique, et les sels correspondants

(ii) des composés azotés hétérocycliques de formule (IVb) :

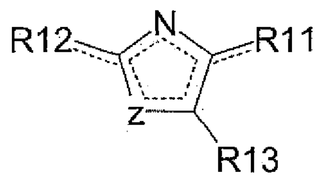

(IVb)

dans laquelle :

les liaisons - - - peuvent signifier une simple liaison ou une double liaison, étant entendu que 2 liaisons contigües ne peuvent pas être des doubles liaisons, les hétérocycles azotés pouvant être saturés, insaturés ou aromatiques ;
$R_{11}$ désigne un atome d'oxygène (sous forme de carbonyle) ou un radical $-CH_2-CH(NH_2)-CO_2H$ ou un groupe -COOH ;
$R_{12}$ désigne un atome d'hydrogène ou un atome d'oxygène (sous forme de carbonyle) ;
$R_{13}$ désigne un atome d'hydrogène ou un radical $-NH-CO-NH_2$ ;
Z désigne un atome d'azote -N= ou un radical $-N(R_{14})-$ dans lequel $R_{14}$ désigne un atome d'hydrogène, ou un radical $-CH_2-CH(NH_2)-CO_2H$ , ou un radical -CH2-;
et les sels correspondants ;

(iii) des composés azotés de formule (Va) :

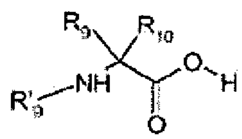

(Va)

dans laquelle :

- Rg représente un atome d'hydrogène ou un radical $C_1$-$C_6$ alkyle linéaire ou ramifié, éventuellement substitué par un ou plusieurs groupes identiques ou différents choisis parmi des radicaux hydroxyle, hydroxycarbonyle, thiol, ($C_1$-$C_4$)-alkylthio, amido, amino ou guanidine, ou un radical phényle, éventuellement substitué par un ou plusieurs hydroxyles ;
- $R'_9$ représente un hydrogène, un radical $C_1$-$C_4$ alkyle, ou un radical benzyle non substitué, ou un radical $C_2$-$C_{16}$ acyle ; $R'_9$ représente préférablement un atome d'hydrogène ;
- $R_{10}$ représente un hydrogène ou un radical $C_1$-$C_4$ alkyle ; $R_{10}$ représente préférablement un atome d'hydrogène,

et les sels correspondants
(iv) des aminopolymères
étant entendu que, lorsque le composé azoté est un acide aminé, alors la poudre colorée ne contient pas d'acide tannique ni de dérivé de gallotannin correspondant, ni les mélanges correspondants.

**2.** Poudre selon la revendication précédente, **caractérisée en ce que** 1'anthacyanine est un composé de formule (I) :

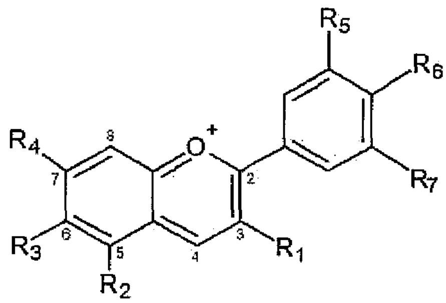

dans laquelle $R_1$, $R_2$ et $R_4$ désignent indépendamment H, OH, OMe, un motif sucre ou un motif sucre acylé, dont le groupe acyle contient 2 à 15 atomes de carbone, étant entendu qu'au moins un des radicaux $R_1$, $R_2$ et $R_4$ désigne un motif sucre ou un motif sucre acylé, et $R_3$, $R_5$, $R_6$ et $R_7$ désignent indépendamment H, OH or OMe.

**3.** Poudre selon l'une ou l'autre des revendications précédentes, **caractérisée en ce que** l'anthocyanine est dérivée de matière végétale choisie parmi la carotte noire, la baie de sureau, l'hibiscus, le maïs violet, la pomme de terre noire, le choux rouge, l'oignon rouge, la pomme de terre violette, le raisin noir, la canneberge, la fraise, la framboise, l'aronia, le soja noir, le cassis, le radis, la groseille à maquereau, la myrtille, la cerise, l'aubergine et le riz noir.

**4.** Poudre selon l'une quelconque des revendications précédentes, **caractérisée en ce que** l'anthocyanine est présente dans la poudre colorée en une teneur dans la plage de 0, 05 % à 50 % en poids, par rapport au poids total de matière sèche de la poudre colorée, préférablement dans la plage de 0,3 % à 30 % en poids, préférentiellement dans la plage de 5 % à 30 % en poids, et plus préférentiellement dans la plage de 10 % à 30 % en poids.

**5.** Poudre selon l'une quelconque des revendications précédentes, **caractérisée en ce que** le métal complexé est l'aluminium.

**6.** Poudre selon l'une quelconque des revendications 1 à 4, **caractérisée en ce que** le métal complexé est dérivé de sels choisis parmi le sulfate d'aluminium, le sulfate d'ammonium et d'aluminium, l'acétate d'aluminium, le chlorure d'aluminium, le chlorhydrate d'aluminium, l'acétate de zinc, le gluconate de zinc, le chlorure de zinc, le sulfate de zinc ; et des mélanges correspondants, et préférablement choisis parmi le sulfate d'ammonium et d'aluminium, le chlorure d'aluminium, le sulfate de zinc, et des mélanges correspondants.

**7.** Poudre selon l'une quelconque des revendications précédentes, **caractérisée en ce que** le métal complexé est présent à raison d'un rapport pondéral d'ions métalliques/anthocyanine dans la plage de 0,01/1 à 10/1, préférablement dans la plage de 0,05/1 à 5/l.

**8.** Poudre selon l'une quelconque des revendications précédentes, **caractérisée en ce que** le composé azoté est un composé de formule (IVa) ou (IVb) et est choisi parmi le nicotinamide adénine diphosphate, le sel d'adénosine triphosphate disodique, la pyridoxine, l'adénosine diphosphate, la théobromine, l'adénosine, la thymidine, la guanine, la cytidine, l'uridine, la caféine, la xanthine, l'acide nicotinique, la trigonelline, la paraxanthine, l'adénine, l'histidine, l'allantoïne, le tryptophane et la proline.

**9.** Poudre selon l'une quelconque des revendications 1 à 7, **caractérisée en ce que** le composé azoté est un composé de formule (Va) et est choisi parmi la guanidine (notamment sous forme de chlorhydrate), la lysine, la N-lauroyllysine et l'arginine.

**10.** Poudre selon l'une quelconque des revendications 1 à 7, **caractérisée en ce que** le composé azoté est un aminopolymère choisi parmi :

- des poly(($C_2$-$C_5$)-alkyléneimine)s ;
- une poly(allylamine) ;
- des polyvinylamines et des copolymères correspondants, notamment avec des vinylamides ;
- un aminodextrane ;
- des copolymères vinylamine/alcool vinylique ;

- des polymères d'acrylamidopropylamine ;
- des chitosanes.

**11.** Poudre selon l'une quelconque des revendications 1 à 7 et 10, **caractérisée en ce que** le composé azoté est une polylysine.

**12.** Poudre selon l'une quelconque des revendications 1 à 7, **caractérisée en ce que** le composé azoté est choisi parmi le nicotinamide adenine diphosphate, la pyridoxine, le sel d'adénosine triphosphate disodique, l'adénosine diphosphate, la théobromine, l'adénosine, la thymidine, la guanine, la cytidine, l'uridine, la caféine, la xanthine, l'allantoïne, l'acide nicotinique, la trigonelline, la paraxanthine, l'histidine, la guanidine (notamment sous forme de chlorhydrate) , la lysine, une polylysine, la N-lauroyl-lysine, l'arginine, l'adénine ; de préférence choisi parmi la pyridoxine, le sel d'adénosine triphosphate disodique, l'adénosine, la caféine, l'allantoine, la trigonelline, l'histidine, une polylysine, la N-lauroyl-lysine, l'arginine, l'adénine ; plus préférentiellement choisi parmi la pyridoxine, le sel d'adénosine triphosphate disodique, l'adénosine, l'allantoine,la trigonelline, l'histidine, une polylysine, l'arginine et l'adénine.

**13.** Poudre selon l'une quelconque des revendications 1 à 7, **caractérisée en ce que** le composé azoté est choisi parmi la pyridoxine, le sel d'adénosine triphosphate disodique, l'adénosine, l'allantoïne, l'histidine et, l'adénine.

**14.** Poudre selon l'une quelconque des revendications 1 à 7, **caractérisée en ce que** le composé azoté est choisi parmi le sel d'adénosine triphosphate disodique et l'histidine.

**15.** Poudre selon l'une quelconque des revendications 1 à 7, **caractérisée en ce que** le composé azoté est le sel d'adénosine triphosphate disodique.

**16.** Poudre selon l'une quelconque des revendications 1 à 7, **caractérisée en ce que** le composé azoté est présent à raison d'un rapport pondéral de composé azoté/anthocyanine dans la plage de 1/10 à 10/1, préférablement dans la plage de 1/3 à 3/1 en poids, préférentiellement dans la plage de 1/2 à 2/1 en poids.

**17.** Poudre selon l'une quelconque des revendications précédentes, **caractérisée en ce qu'**elle possède une couleur bleu et un angle de teinte compris entre 210° et 325°, et préférablement entre 250° et 300°.

**18.** Composition cosmétique comprenant, dans un milieu physiologiquement acceptable, une poudre colorée selon l'une quelconque des revendications précédentes.

**19.** Procédé cosmétique de traitement de matières kératiniques, comprenant l'application aux matières kératiniques d'une poudre colorée selon l'une des revendications 1 à 17 ou d'une composition selon la revendication 18.

**20.** Procédé selon la revendication précédente, **caractérisé en ce qu'**il s'agit d'un procédé pour le maquillage de matières kératiniques.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014029842 A
- FR 2994693


### Non-patent literature cited in the description


- **RAYMOND BROUILLARD ; ANDRE FOUGEROSSE.** *Le mystère de la couleur des fleurs, des fruits ... et du vin!" ("The mystery of the colour of flowers, fruit...and wine!),* 18 February 2008, http://radium.net.espci.fr/esp/CONF/2008/C08_02/conf02_2008.htm
- **JIN-MING KONG et al.** *Phytocomposé,* 2003, vol. 64, 923-933
- **A CASTAÑEDA-OVANDO et al.** *Food Compose,* 2009, vol. 113, 859-871
- **F.J. FRANCIS.** Colorants. Eagan Press, 1999, 56
- CIE technical report. Colorimetry. 1986